(19) Europäisches Patentamt
European Patent Office
Office européen des brevets

(11) **EP 2 605 734 B1**

(12) **EUROPEAN PATENT SPECIFICATION**

(45) Date of publication and mention
of the grant of the patent:
**05.04.2017 Bulletin 2017/14**

(51) Int Cl.:
***A61F 5/443*** *(2006.01)* ***A61F 5/445*** *(2006.01)*
***A61L 24/04*** *(2006.01)*

(21) Application number: **11746157.4**

(22) Date of filing: **16.08.2011**

(86) International application number:
**PCT/DK2011/050309**

(87) International publication number:
**WO 2012/022352 (23.02.2012 Gazette 2012/08)**

(54) **A COLLECTING DEVICE**

SAMMELVORRICHTUNG

DISPOSITIF COLLECTEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.08.2010 DK 201070361**

(43) Date of publication of application:
**26.06.2013 Bulletin 2013/26**

(73) Proprietor: **Coloplast A/S**
**3050 Humlebæk (DK)**

(72) Inventors:
• **KLEIN, Charlotte**
**DK-2700 Broenshoej (DK)**
• **BUUS, Hasse**
**DK-3050 Humlebaek (DK)**
• **STROEBECH, Esben**
**DK-2970 Hoersholm (DK)**
• **KONGEBO, Tom Bjarke**
**DK-3050 Humlebaek (DK)**

(56) References cited:
**WO-A1-2009/006901 WO-A1-2009/127208
WO-A1-2010/066254**

## Description

### Field of the Invention

The present invention relates to a collecting device for attachment to human skin and for collecting bodily waste e.g. an ostomy device.

### Background

Collecting devices for collecting bodily waste, ostomy appliances, wound or fistulae drainage bandages or devices for collecting urine are usually in the form of a receptacle, e.g. a bag, pouch or tube for receiving the waste, connected to an adhesive wafer that can be attached to the skin of the patient. The wafer is typically in the form of a backing layer coated on the skin-facing surface with an adhesive layer and the wafer may further be provided with an aperture for accommodating the body opening. The size and shape of said aperture can often be adapted individually to fit the anatomy of the patient.

One of the crucial parts of such devices is the adhesive wafer. The wafer should be able to fit leak proof around the body opening, have good adherence to the skin without unintended detachment from the skin, but at the same time be easy to remove without damaging the skin. Furthermore, the wafer should be able to follow the movements of the body and be comfortable to wear.

When designing a skin adhesive, one of the major issues is to keep the skin relatively dry underneath the adhesive to prevent maceration. Maceration occurs when the skin is unable to get rid of moisture from transpiration and outlet from a body opening. This may result in degradation of the skin's barrier function as well as bad adhesion of the device to the skin.

Usually, skin adhesive keep the skin dry by absorbing moisture. Absorbing particles or hydrocolloids (HC) are mixed into an adhesive matrix in order to absorb moisture from the skin and thereby the skin is kept relatively dry. The high content of hydrocolloids makes this type of adhesive relatively stiff and inflexible. This technique is well known in the art and forms the basis for most ostomy adhesives that are commercially available see, e.g. US Patent No. 4,192,785. Alternatively, skin adhesive can be permeable, having a high moisture vapour transmission rate, MVTR, whereby the adhesive is keeping the skin dry by permeating the moisture through the adhesive layer instead of absorbing the moisture. This type of adhesive is flexible and soft. The flexible adhesive wafer made by this type of adhesive is thin, typically less than 100μm.

A problem with these thin flexible adhesives is their tendency to wrinkle and adhere to themselves which make them difficult to handle. In order to make such thin adhesive wafer easier to apply to the skin of a person, they are provided with a temporary stiffening layer which is removed once the wafer has been applied. It is very difficult to apply thin flexible adhesive wafers without such a stiffening layer. In one example, the thin adhesive is placed on a non-wowen material which to a certain extent is capable of supporting itself. Other examples may require paper frames and optionally split release liners. Split release liners are used because of the high initial tack of the adhesive. The split release liners allow the wafer to be positioned in the correct position one step at the time before removing the whole of the release liner from the wafer, thus providing the user with a higher degree of freedom in the application process.

WO2009/127208 relates to body waste collecting device.

WO 2010/074640 describes a device for facilitating application of an adhesive plastic film to the skin. The plastic film is a component in a wound dressing or other medical article comprising a stiffening layer removably attached to said film. It has now surprisingly been found that a permeable flexible adhesive wafer can be made without a stiffening layer and still avoid the tendency to wrinkle and adhere to itself.

### Summary of the invention

The present invention relates to a body waste collecting device comprising a collecting pouch and an adhesive wafer for attachment to the body, said adhesive wafer comprises a backing layer, a first adhesive being a hydrocolloid pressure sensitive adhesive, a second adhesive and a release liner, wherein said second adhesive comprises a polar plasticising oil or a combination of polar plasticising oils in the content of above 10% (w/w) of the final second adhesive, and at least one polar polyethylene copolymer, wherein the content of the polyethylene copolymer is 10-50% (w/w) of the final second adhesive, the polyethylene copolymer has a melt flow index below 2 g/10min (190°C/21.1 N), and wherein the thickness of the second adhesive is 300-700 μm.

### Detailed description of the present invention

The aim of the invention is to provide a body waste collecting device that includes a first adhesive and a second adhesive, wherein said second adhesive comprises a polar plasticising oil or a combination of polar plasticising oils in the content of above 10% (w/w) of the final second adhesive, and at least one polar polyethylene copolymer, wherein the content of the polyethylene copolymer is 10-50% (w/w) of the final second adhesive, the polyethylene copolymer has a melt flow index below 2 g/10min (190°C/21.1 N), and wherein the thickness of the second adhesive is 300-700 $\mu$m.

By body waste collecting device is meant a device being able to collect and keep the output in a collecting item for a predefined time. The holding in place of the device may be obtained by a skin adhesive and the collection may be obtained by a bag.

In an embodiment of the invention, a body waste collecting device comprises a collecting pouch and an adhesive wafer for attachment to the body, said adhesive wafer comprises a backing layer, a first adhesive being a hydrocolloid pressure sensitive adhesive, a second adhesive and a release liner, wherein said second adhesive comprises a polar plasticising oil or a combination of polar plasticising oils in the content of above 10% (w/w) of the final second adhesive, and at least one polar polyethylene copolymer, wherein the content of the polyethylene copolymer is 10-50% (w/w) of the final second adhesive, the polyethylene copolymer has a melt flow index below 2 g/10min (190°C/21.1 N), and wherein the thickness of the second adhesive is 300-700 $\mu$m.

The polyethylene copolymer based adhesive system does not absorb the moisture but rather permeates the water away from the skin surface. The adhesion is usually obtained by wetting and affinity to the skin.

It has now surprisingly been found that using this polyethylene copolymer based adhesive as the second adhesive in the thickness of 300-700 $\mu$m makes a flexible and soft adhesive wafer which can be handled without a stiffening layer.

According to an embodiment of the invention, the thickness of the second adhesive is 400-600 $\mu$m, preferably 500 $\mu$m.

The thickness of the second adhesive may be uniform or essentially uniform. By uniform is meant that the second adhesive has the same thickness or essentially the same thickness.

By the thickness of the second adhesive is meant the maximum thickness of the second adhesive layer.

According to another embodiment, the thickness of the second adhesive may vary.

In one embodiment the thickness of the second adhesive may vary from 450 to 500 $\mu$m. Accordingly, in this embodiment the thickness of the second adhesive is said to be 500 $\mu$m.

In an embodiment the thickness of the second adhesive may vary according to a pattern.

The adhesive wafer according to the invention can be handled in a similar manner as the conventional hydrocolloid pressure sensitive adhesive system, however still maintaining the characteristics of flexibility and softness.

When applying the adhesive wafer in a simple application process without split release liners and paper frame, the initial adhesion or tack is another parameter of importance. The tack has to be sufficient to enable the product to adhere to the user's body upon application, but the tack should also be low enough to allow the user to re-applicate and adjust the product, if positioned incorrectly.

The second adhesive according to the invention fulfills this criterion.

If the tack of the adhesive is too weak, the adhesive cannot adhere properly to the skin. However, if the tack is too high, the adhesive will stick to the surface when trying to remove it, finally resulting in breaking the adhesive and leaving residues on the skin or causing skin stripping of the body. Neither option is desirable.

PSA adhesive types can be formulated into different tackiness. Tack depends of various factors like, type of substrate to be adhered on, aggressiveness, oil or resin content and type, rate of filling, chemistry etc.

General tack of state of the art PSA adhesives used in ostomy care is formulated to be relatively low as a low tack is needed in order to be able to give a right position to the skin when a colleting device like an ostomy bag is applied to the abdomen. The tack desired should be that high that it stays on the skin when applied, but will not bond to the skin at first skin contact. Adhesion to the skin will evolve with time and is accelerated if pressure is applied.

High tack adhesives that have acceptable peel force when removed from the skin do not have the feature of an easy application, as the high tack tends to bond to the skin at first skin contact.

The tack of the second adhesive according to the invention is similar to the tack of the conventional hydrocolloid pressure sensitive adhesive.

The second adhesive according to the invention is thicker than the flexible soft adhesives known in the art. Thus, the polyethylene copolymer based adhesive according to the invention can support itself. As the tack will increase with the thickness of the adhesive, some of the flexible soft adhesives known in the art will be too tacky resulting in the problems mentioned above.

Other permeable adhesives of the flexible soft adhesive type known in the art, for example an acrylate based adhesive, cannot be used in a thicker version due to loss of permeability when the adhesive layer is too thick. These adhesives are therefore used only as thin layers which necessitate the stiffening layer for application purposes.

The second adhesive of the invention comprises a polar plasticising oil or a combination of polar plasticising oils in the content of above 10% (w/w) of the final second adhesive, and at least one polar polyethylene copolymer, wherein the content of the polyethylene copolymer is 10-50% (w/w) of the final second adhesive, the polyethylene copolymer has a melt flow index below 2 g/10min (190°C/21.1 N).

According to one embodiment of the invention, the second adhesive is produced by mixing a polar plasticising oil or a combination of polar plasticising oils in the content of above 10% (w/w) of the final second adhesive, and at least one polar polyethylene copolymer, wherein the content of the polyethylene copolymer is 10-50% (w/w) of the final second adhesive, the polyethylene copolymer has a melt flow index below 2 g/10min (190°C/21.1 N).

In an embodiment of the invention, the final second adhesive in continuous form exhibits a moisture vapour transmission rate of at least 100 g/m$^2$/day for a 150 $\mu$m adhesive sheet when measured according to MVTR Test Method as defined in the description.

The primary polymers used in the second adhesive are ethylene copolymers. The copolymer should contain a considerable amount of a polar component to get high water permeability. Preferably, the ethylene parts of the copolymer can form crystalline areas that ensure the cohesive strength of the adhesive.

In one embodiment of the invention, the polar polyethylene copolymer is selected from the group consisting of ethylene vinyl acetate, ethylene vinyl acetate carbon monoxide, ethylene butyl acetate, ethylene vinyl alcohol, ethylene butyl acrylate, ethylene butyl acrylate carbon monoxide, and combinations thereof.

The polar polyethylene copolymer is preferably ethylene vinyl acetate.

The adhesive composition comprising ethylene vinyl acetate may suitably be an adhesive known in the art, such as the adhesive composition disclosed, for example in International Patent Application No. PCT/DK2008/050146.

By polar polymers is meant polymers with water transmission above 50 g/m$^2$/day for a 150 $\mu$m film when measured according to MVTR Test Method.

In an embodiment of the invention, the ethylene vinyl acetate has a content of at least 40% (w/w) vinyl acetate preferably with 40-80% (w/w) vinyl acetate.

The second adhesive should fulfill the Dahlquist's criterion. Preferably, the modulus should be below 100 000 Pa and for very soft, skin friendly and comfortable adhesive the modulus (G') could be as low as 1-30 kPa measured by DMA at 32°C and 1 Hz.

It is of great importance, that the second adhesive is as soft as possible to ensure a skin friendly material that is comfortable to wear. To get a soft material, the polymer content should be as low as possible. The maximum polymer content of the polar polyethylene copolymer should not exceed 50% (w/w) of the final second adhesive.

Preferably, the polar polyethylene copolymers used in the second adhesive should have a molecular structure at a level that results in a melt flow index (MFI) below 2 g/10min (190°C/21.1 N). The melt flow index can be measured by the methods given in ISO 1133 and ASTM D1238.

The advantage of using a polymer with high molecular weight and low MFI is that the high molecular weight polymer can ensure a sufficient high cohesive strength to the second adhesive.

By the final second adhesive is meant the final adhesive compound after mixing the constituents.

By the content of the final second adhesive is meant the percentage in weight of the ingredient in relation to the total weight of the ingredients used in the second adhesive.

In an embodiment of the invention, the content of the polar polyethylene copolymer is 10-45% (w/w) of the final second adhesive preferably 15-30%.

In another embodiment of the invention, the polar polyethylene copolymer has a molecular weight above 250,000 g/mol.

In one embodiment of the present invention, the second adhesive comprises a polar plasticising oil or a combination of polar plasticising oils in the content of 20-70% (w/w) of the final second adhesive preferably 30-65%.

Polar oils, which may be used in the invention, will generally be those that have good solubility in the polar domains of the polymer, i.e. provide softness without sacrificing too much tensile strength of the polymer. Oils that can support good water vapour permeability are preferred, a 50:50 mix of polymer and oil should have a moisture vapour transmission rate of at least 100 g/m$^2$/day. Examples of such oils are vegetable and animal oils and derivatives thereof. Preferred polar oils are esters, ethers and glycols and particularly preferred is Poly Propylene Oxide, e.g. alpha-butoxy-polyoxypropylene.

The second adhesive should preferable contain about or more than 40% plasticising oil to get the optimal softness and skin friendliness.

In one embodiment of the present invention, the second adhesive comprising a polar plasticising oil wherein the polar plasticising oil is selected from the group of liquid rosin derivatives, aromatic olefin oligomers, vegetable and animal oils and derivatives, preferable polar oils are esters, ethers and glycols and particularly preferred is poly propylene oxides such as alpha-butoxy-polyoxypropylene.

Furthermore, polypropylene oxide oil contributes to a high permeability of the second adhesive.

Some of the second adhesives according to the invention contain a minor amount of additional polymer besides the main polymer giving cohesion. This or these additional polymers are added to give tack. These additional polymers are optional and not necessary for all purposes.

In one embodiment of the invention, the second adhesive further comprises a low molecular weight polymer, i.e. MFI >2.

The addition of a low Mw polymer to the second adhesive may be an advantage when a lot of moist is present between the second adhesive and the skin.

Preferably the total polymer content, including polar polyethylene copolymer and additional polymers (not including oils, tackifier resin etc), should not exceed 50% (w/w) of the final second adhesive.

Additional components may be added to the second adhesive such as tackifier resin, plasticisers and wax.

In one embodiment of the invention, the second adhesive further comprises a tackifying resin such as natural, modified or synthetic resins preferably polar resins such as rosins, rosin esters, hydrogenated rosins, hydrogenated rosin esters, and derivatives of such polar resins or pure aromatic monomer resins. Tackifying resins can be added to control tack in the adhesives, i.e. reduce moduli and increase glass transition temperature.

The content of the tackifying resin is 0-40% (w/w) of the final second adhesive. Preferably the adhesive is substantially free of resin. When the second adhesive is containing resin, the content of the tackifying resin is preferably 0.1-40% (w/w) of the final second adhesive and more preferably 10-20% (w/w) of the final second adhesive.

In one embodiment of the present invention, the second adhesive comprises polar plasticising oils and resin in the content of above 50% (w/w) of the final second adhesive.

In one embodiment of the invention, the second adhesive further comprises an additional plasticiser selected from the group of mineral oil, citrate oil, paraffin oil, phthalic acid esters, adipic acid esters (e.g. DOA), and liquid or solid resin.

In another embodiment of the invention, the second adhesive further comprises a polyethylene wax.

Other ingredients may be added for auxiliary benefits. This could be antioxidants and stabilisers, fillers for rheology modification or active components like vitamin E or ibuprofen.

In another embodiment of the invention, the second adhesive further comprises other ingredients selected from the group of antioxidants, stabilisers, fillers, pigments, flow modifiers, and active ingredients.

In one preferred embodiment of the invention, the second adhesive comprises polar active ingredients.

It may be advantageous that the second adhesive comprises absorbent particles. According to an embodiment of the invention, the second adhesive comprises absorbent particles.

In one embodiment of the invention, the content of the absorbent particles is 0.1-40% (w/w) of the final second adhesive preferably 10-30%.

The particles may be absorbent particles such as hydrocolloids, microcolloids or super absorbers in order for the composition to absorb moisture from skin.

According to an embodiment of the invention, the absorbent particles are selected from hydrocolloids, microcolloids and super absorbers.

Microcolloid particles are well-known in the art e.g. from International Publication No. WO 02/066087, which discloses adhesive compositions comprising microcolloid particles. The microcolloid particles may have a particle size of less than 20 microns.

According to an embodiment of the invention, the first adhesive comprises absorbent particles.

The first adhesive may comprise 1 to 60 % w/w of hydrocolloid (HC) or super absorbent particles (SAP) particles, more preferred 30 to 60% w/w particles.

According to an embodiment of the invention, the first adhesive is located adjacent to the body waste source, usually an ostomy or a fistula.

By located adjacent to is meant that the subject is located close to or even in contact with the other subject.

The first adhesive is a hydrocolloid pressure sensitive adhesive.

A standard and conventional hydrocolloid (HC) pressure sensitive adhesive is a skin friendly adhesive that is capable of adhering to the skin, handling perspiration by absorbing the moist and being removable from the skin without essential damage.

A typical pressure sensitive adhesive is based on a polyisobutylene, PIB (adhering substance) and carboxy methyl cellulose, CMC (absorbing media).

A typical HC pressure sensitive adhesive, as used today, is a highly filled hydrocolloid system with a matrix of polymers that exhibit flow as well as cohesive properties. The hydrocolloids absorb the moisture from perspiration and output from the body and the polymers adhere to the skin through skin polymer affinity and the polymers flow into the small cavities of the skin. The cohesion of the adhesive ensures that erosion is minimised and that the wafer is removed in one piece and that minimal adhesive residues are left on the skin.

In an embodiment of the invention, the first adhesive is located on the skin-facing surface of the second adhesive.

By the skin-facing surface of the adhesive is meant the side adhering to the skin.

By located is meant where the adhesive is placed on the collecting device and specifies the position of the second adhesive or the first adhesive on the collecting device.

In another embodiment of the invention, the second adhesive is located on the pouch-facing surface of the adhesive wafer.

By the pouch-facing surface or non-skin-facing surface is meant the side of the adhesive or backing pointing away from the skin (non-bonding side).

According to one embodiment of the invention, the second adhesive is located at the outer rim of the adhesive wafer.

By the outer rim portion of the adhesive wafer is meant the portion essentially outside the welding zone or coupling. This portion is less exposed to faeces but has to handle perspiration as well as mechanical exposure due to movements and pull from the bag.

By the inner rim portion of the adhesive wafer is meant the portion in the perianal, peristomal or fistal area. This area of the adhesive is exposed to faeces or exudates as well as perspiration. The properties of the inner rim portion depend on wear time, output type and anatomy among others.

According to an embodiment of the invention, the second adhesive is in the form of a ring.

By an adhesive ring is meant an adhesive, essentially surrounding the body opening, that needs to be drained or a ring covering the outer or inner rim of the adhesive wafer.

According to one embodiment of the invention, the second adhesive is part of a ring such as a half ring covering 180 degrees of a circle.

In an embodiment of the invention, the thickness of the second adhesive may vary according to a pattern.

According to one embodiment, the thickness of the second adhesive is thicker in a ring covering the outer rim of the adhesive wafer, whereas a ring of the second adhesive covering a part of the adhesive wafer from the outer rim towards the inner rim of the adhesive wafer is thinner than the ring covering the outer rim of the adhesive wafer.

In one embodiment, the ring of the second adhesive covering the outer rim is 2-10 mm broad, preferably 3-5 mm broad.

According to one embodiment, the ring of the second adhesive covering a part of the adhesive wafer from the outer rim towards the inner rim of the adhesive wafer is 10-20 mm broad.

The thicker part of the second adhesive should be large enough to give the desired stiffness of the device. Thus, the adhesive can support itself and it is possible to avoid additional stiffening means for the medical device.

In an embodiment, the ring of the second adhesive covering the outer rim of the adhesive wafer has a thickness of 300-700 $\mu$m, preferably 400-600 $\mu$m, more preferably 500 $\mu$m.

According to one embodiment, the ring of the second adhesive covering a part of the adhesive wafer from the outer rim towards the inner rim of the adhesive wafer has a thickness of 100-300 $\mu$m, preferably 200 $\mu$m.

According to an embodiment of the invention, the first adhesive only covers a part of the second adhesive on the skin-facing surface.

According to an embodiment of the invention, the second adhesive partly covers the adhesive wafer.

According to another embodiment of the invention, the second adhesive partly covers the skin-facing surface of the first adhesive.

In one embodiment of the invention, the release liner consists of one release liner.

By using a single release liner, the application process of the adhesive wafer is simplified.

The backing layer of the device of the present invention is preferably in the form of a polymer film, coating, laminate, textile or non-woven. The backing layer is preferably a highly flexible film, being strong enough for attachment of e.g. couplings and/or pouch and for removing the device in one piece, but soft enough to follow the movements of the body.

A preferred backing layer is a polyurethane film.

According to an embodiment of the invention, the backing layer is non-wowen.

Preferably, the backing layer has thermoplastic elements that enable welding of e.g. a pouch or coupling ring to the adhesive wafer. Preferred thickness of the backing layer is between 10-60 $\mu$m in order to maintain the softness of the adhesive wafer.

According to an embodiment of the invention, the body waste source is a stoma.

According to another embodiment of the invention, the body waste source is a fistula.

According to another embodiment of the invention, the body waste source is an anus.

By a body waste source is meant a natural or artificial body opening such as an anus, a stoma or a fistula.

According to an embodiment of the invention, the collecting device is an ostomy appliance.

According to another embodiment of the invention, the collecting device is a faecal collecting device.

According to another embodiment of the invention, the collecting device is a fistula collecting device.

According to an embodiment of the invention, the collecting pouch is detachable.

According to another embodiment of the invention, the collecting pouch is integrated with the wafer.

The collecting pouch may be detachable from the adhesive wafer by a coupling system or the pouch and the wafer may be integrated with the wafer, e.g. by welding. The two versions are known as one piece or two-piece appliances for ostomy.

**Experimental**

Laboratory methods

Method 1: Determination of moisture vapour transmission rate (MVTR).

MVTR was measured in grams per square meter ($g/m^2$) over a 24 hours period using an inverted cup method.
A container or cup that was water and water vapour impermeable having an opening was used. 20 ml saline water (0.9%NaCl in demineralised water) was placed in the container and the opening was sealed with the test adhesive film. The container was placed into an electrically heated humidity cabinet and the container or cup was placed upside down, such that the water was in contact with the adhesive. The cabinet was maintained at 37°C and 15% relative humidity (RH). The weight loss of the container was followed as a function of time. The weight loss was due to evaporation of water vapour transmitted through the adhesive film. This difference was used to calculate Moisture vapour transmission rate or MVTR. MVTR was calculated as the weight loss per time divided by the area of the opening in the cup ($g/m^2/24h$). The MVTR of a material was a linear function of the thickness of the material. Thus, when reporting MVTR to characterise a material, it was important to inform the thickness of the material which MVTR was reported. We used $150\mu m$ as a reference. If thinner or thicker samples were measured, the MVTR was reported as corresponding to a $150\mu m$ sample. Thus a $300\mu m$ sample with a measured MVTR of $10g/m^2/24h$ was reported as having MVTR=$20g/m^2/24h$ for a $150\mu m$ sample because of the linear connection between thickness of sample and MVTR of sample.
Finally, we noted that by using this method, we introduced an error by using a supporting PU film. Utilising the fact, that the adhesive/film laminate was a system of two resistances in series eliminated the error. When the film and the adhesive are homogeneous, the transmission rate may be expressed as:

$$1/P(measured) = 1/P(Film) + 1/P(Adhesive).$$

Hence, by knowing the film permeability and thickness of the adhesive, it was possible to calculate the true permeability of the adhesive (P(Adhesive)) using the following expression:

$$P(adhesive) = d(Adhesive)/150micron * 1/(1/P(measured) - 1/P(Film))$$

where d(Adhesive) was the actual measured thickness of the adhesive and P(Film) was the MVTR of the film without any adhesive on and P(measured) was the actual measured MVTR.

Method 2: DMA and determination of G' and $\tan(\delta)$

The parameters G' and $\tan(\delta)$ were measured as follows: The adhesives were pressed into a plate of 1 mm thickness. A round sample of 25 mm in diameter was cut out and placed in a RheoStress RS600 rheometer from Thermo Electron. The geometry applied was parallel plates 25 mm and the deformation was fixed at 1% to ensure that measurements were in the linear regime. The measurements were carried out at 32°C.

Probe tack experiments

Using ASTM D2979-01 principals for the measurements of tack of Pressure Sensitive adhesives (PSA) the following test method was used.
A cylinder probe, cleaned with ethanol and dried for 1 min, was placed in an Instron 5564 with 500 N load cell. The probe had a contact surface of app 28 $mm^2$ with a diameter of 6 mm and was coated with a PTFE coating.
The PSA was adhered to an object glass with a double sided adhesive.
The probe was pressed into the PSA adhesive with a speed of 0.5 mm/min until a load of 2 N was obtained. The probe was held in place for 2 s and was then removed from the PSA sample with a speed of 0.5 mm/min until no joining. The energy in mJ was measured.
Mean of 8 measurements were used to express the tack of the PSA sample results.

| Sample no. | Tack energy mJ | SD | Thickness mm | Comment |
|:---:|:---:|:---:|:---:|:---:|
| 1 | 1.3 | 0.11 | 0.9 | Easy to adjust to skin |
| 2 | 0.9 | 0.15 | 1.0 | Easy to adjust to skin |
| 3 | 2.0 | 0.30 | 1.0 | Easy to adjust to skin |
| 4 | 5.1 | 0.24 | 0.7 | Difficult to adjust to skin |

1. Adhesive according to the invention
2. Standard ostomy care hydrocolloid (SenSura) adhesive (EP1981554)
3. Standard ostomy care hydrocolloid (Assura, Tera) adhesive (EP1198261)
4. Silyl propopylene oxide (according to EP2120812) adhesive

The level of tack from 0.9-2.0 mJ at two commercially available adhesives had the right tack in order to obtain an easy application of the device. In the case of a high tack adhesive where the tack was 5.1 mJ an easy application to the skin was not possible as instant bonding restricted the possibility of adjusting the device to a perfect position.

The tack according to the invention was 1.3 mJ, which was in the same range of tack level as the commercially available HC containing PSA's. The tack should not exceed 3 mJ before problems with applications will occur. The perception of tack to the skin is very subjective and an easy application of an ostomy product depends on various factors like, skills, anatomy, skin type etc.

## Claims

1. A body waste collecting device comprising

    - a collecting pouch
    - an adhesive wafer for attachment to the body, comprising

        - a backing layer
        - a first adhesive being a hydrocolloid pressure sensitive adhesive,
        - a second adhesive
        - a release liner,

    wherein said second adhesive comprises a polar plasticising oil or a combination of polar plasticising oils in the content of above 10% (w/w) of the final second adhesive, and at least one polar polyethylene copolymer, wherein the content of the polyethylene copolymer is 10-50% (w/w) of the final second adhesive, the polyethylene copolymer has a melt flow index below 2 g/10min (190°C/21.1 N), and wherein the thickness of the second adhesive is 300-700 $\mu$m.

2. The collecting device according to claim 1, wherein the final second adhesive in continuous form exhibits a moisture vapour transmission rate of at least 100 g/m$^2$/day for a 150 $\mu$m adhesive sheet when measured according to MVTR Test Method as defined in the description.

3. The collecting device according to any of claims 1-2, wherein the polar polyethylene copolymer is selected from the group consisting of ethylene vinyl acetate, ethylene vinyl acetate carbon monoxide, ethylene butyl acetate, ethylene vinyl alcohol, ethylene butyl acrylate, ethylene butyl acrylate carbon monoxide, and combinations thereof.

4. The collecting device according to claim 3, wherein the polar polyethylene copolymer is ethylene vinyl acetate.

5. The collecting device according to claim 4, wherein the ethylene vinyl acetate has a content of at least 40% (w/w) vinyl acetate preferably with 40-80% (w/w) vinyl acetate.

6. The collecting device according to any of claims 1-5, wherein the content of the polar polyethylene copolymer is 10-45% (w/w) of the final second adhesive preferably 15-30%.

7. The collecting device according to any of claims 1-6, wherein the polar plasticising oil is selected from the group of liquid rosin derivatives, aromatic olefin oligomers, vegetable and animal oils and derivatives, preferable polar oils are esters, ethers and glycols and particularly preferred is polypropylene oxide such as alpha-butoxy-polyoxypropylene.

8. The collecting device according to any of claims 1-7, wherein the second adhesive further comprises a tackifying resin such as natural, modified or synthetic resins preferably polar resins such as rosin esters and derivatives thereof or pure aromatic monomer resins.

9. The collecting device according to any of the preceding claims, wherein the second adhesive comprises absorbent particles.

10. The collecting device according to any of the preceding claims, wherein the first adhesive is located adjacent to the body waste source.

11. The collecting device according to any of the preceding claims, wherein the second adhesive is located at the outer rim of the adhesive wafer.

12. The collecting device according to any of the preceding claims, wherein the second adhesive is in the form of a ring.

13. The collecting device according to any of the preceding claims, wherein the second adhesive partly covers the adhesive wafer.

14. The collecting device according to any of the preceding claims, wherein the thickness of the second adhesive is 400-600 $\mu$m, preferably 500 $\mu$m.

15. The collecting device according to any of the preceding claims, wherein the collecting device is an ostomy appliance.

**Patentansprüche**

1. Vorrichtung zum Sammeln von Körperabfällen, umfassend

- einen Sammelbeutel
- eine Haftscheibe zur Befestigung am Körper, umfassend

- eine Trägerschicht
- einen ersten Klebstoff, der ein Hydrokolloid-Haftklebstoff ist,
- einen zweiten Klebstoff
- eine Trennschicht,

wobei der zweite Klebstoff ein polares plastifizierendes Öl oder eine Kombination von polaren plastifizerenden Ölen in einem Gehalt von über 10 % (Gew./Gew.) des fertigen zweiten Klebstoffs und wenigstens ein polares Polyethylen-Copolymer umfasst, wobei der Gehalt an dem Polyethylen-Copolymer 10-50 % (Gew./Gew.) des fertigen zweiten Klebstoffs ist, wobei das Polyethylen-Copolymer einen Schmelzflussindex von unter 2 g/10 min (190 °C/21,1 N) aufweist, und wobei die Dicke des zweiten Klebstoffs 300-700 $\mu$m beträgt.

2. Sammelvorrichtung gemäß Anspruch 1, wobei der fertige zweite Klebstoff in kontinuierlicher Form bei Messung nach dem MVTR-Prüfverfahren, wie in der Beschreibung definiert, eine Wasserdampf-Durchlassrate von wenigstens 100 g/m$^2$/Tag bei einer 150$\mu$m-Haftfolie aufweist.

3. Sammelvorrichtung gemäß einem der Ansprüche 1-2, wobei das polare Polyethylen-Copolymer ausgewählt ist aus der Gruppe bestehend aus Ethylenvinylacetat, Ethylenvinylacetat-Kohlenmonoxid, Ethylenbutylacetat, Ethylenvinylalkohol, Ethylenbutylacrylat, Ethylenbutylacrylat-Kohlenmonoxid und Kombinationen davon.

4. Sammelvorrichtung gemäß Anspruch 3, wobei das polare Polyethylen-Copolymer Ethylenvinylacetat ist.

5. Sammelvorrichtung gemäß Anspruch 4, wobei das Ethylenvinylacetat einen Gehalt an Vinylacetat von wenigstens 40 % (Gew./Gew.) aufweist, vorzugsweise von 40-80 % (Gew./Gew.) Vinylacetat.

6. Sammelvorrichtung gemäß einem der Ansprüche 1-5, wobei der Gehalt an dem polaren Polyethylen-Copolymer 10-45 % (Gew./Gew.) des fertigen zweiten Klebstoffs beträgt, vorzugsweise 15-30 %.

7. Sammelvorrichtung gemäß einem der Ansprüche 1-6, wobei das polare plastifizierende Öl ausgewählt ist aus der Gruppe von flüssigen Kolophoniumderivaten, aromatischen Olefinoligomeren, Pflanzen- und Tierölen und -derivaten, wobei bevorzugte polare Öle Ester, Ether und Glykole sind und Polypropylenoxid, wie z. B. alpha-Butoxypolyoxypropylen, besonders bevorzugt ist.

8. Sammelvorrichtung gemäß einem der Ansprüche 1-7, wobei der zweite Klebstoff ferner ein klebrigmachendes Harz umfasst, wie z. B. natürliche, modifizierte oder synthetische Harze, vorzugsweise polare Harze, wie z. B. Kolophoniumester und Derivate davon oder reine aromatische Monomerharze.

9. Sammelvorrichtung gemäß einem der vorstehenden Ansprüche, wobei der zweite Klebstoff Absorbens-Partikel umfasst.

10. Sammelvorrichtung gemäß einem der vorstehenden Ansprüche, wobei der erste Klebstoff benachbart zu der Körperabfall-Quelle angeordnet ist.

11. Sammelvorrichtung gemäß einem der vorstehenden Ansprüche, wobei der zweite Klebstoff an dem äußeren Rand der Haftscheibe angeordnet ist.

12. Sammelvorrichtung gemäß einem der vorstehenden Ansprüche, wobei der zweite Klebstoff in der Form eines Rings vorliegt.

13. Sammelvorrichtung gemäß einem der vorstehenden Ansprüche, wobei der zweite Klebstoff die Haftscheibe teilweise bedeckt.

14. Sammelvorrichtung gemäß einem der vorstehenden Ansprüche, wobei die Dicke des zweiten Klebstoffs 400-600 $\mu$m, bevorzugt 500$\mu$m beträgt.

15. Sammelvorrichtung gemäß einem der vorstehenden Ansprüche, wobei die Sammelvorrichtung eine Stoma-Anwendung ist.

**Revendications**

1. Dispositif de collecte de déchets corporels comprenant

   - une poche de collecte
   - une feuille gaufrée adhésive pour fixation au corps, comprenant
   - une couche de support
   - un premier adhésif étant un adhésif autocollant d'hydrocolloïde
   - un deuxième adhésif
   - une doublure antiadhesive,

   où ledit deuxième adhésif comprend une huile plastifiante polaire ou une combinaison d'huiles plastifiantes polaires à une teneur supérieure à 10 % (m/m) du deuxième adhésif final, et au moins un copolymère de polyéthylène polaire, où la teneur du copolymère de polyéthylène est de 10 à 50 % (m/m) du deuxième adhésif final, le copolymère de polyéthylène a un indice de fluage (MFI) inférieur à 2 g/10 min (190 °C/21,1 N), et où l'épaisseur du deuxième adhésif est 300-700 $\mu$m.

2. Dispositif de collecte selon la revendication 1, dans lequel le deuxième adhésif final sous forme continue présente un taux de transmission de vapeur d'eau d'au moins 100 g/m$^2$/jour pour une couche d'adhésif de 150 $\mu$m mesurée selon la méthode d'essai MVTR comme décrit dans la description.

3. Dispositif de collecte selon l'une quelconque des revendications 1 à 2, où le copolymère de polyéthylène polaire est choisi dans le groupe constitué des éthylène-acétate de vinyle, éthylène-acétate de vinyle-monoxyde de carbone, éthylène-acétate de butyle, éthylène-alcool vinylique, éthylène-acrylate de butyle, éthylène-acrylate de butyle-monoxyde de carbone, et des combinaisons de ceux-ci.

4. Dispositif de collecte selon la revendication 3, où le copolymère de polyéthylène polaire est éthylène-acétate de vinyle.

5. Dispositif de collecte selon la revendication 4, où l'éthylène-acétate de vinyle a une teneur d'au moins 40 % (m/m) d'acétate de vinyle, de préférence avec 40 à 80 % (m/m) d'acétate de vinyle.

6. Dispositif de collecte selon l'une quelconque des revendications 1 à 5, dans lequel la teneur du copolymère de polyéthylène polaire est de 10 à 45 % (m/m) du deuxième adhésif final de préférence de 15 à 30%.

7. Dispositif de collecte selon l'une quelconque des revendications 1 à 6, où l'huile plastifiante polaire est choisie dans le groupe constitué de dérivés de rosine liquide, oligomères d'oléfine aromatique, huiles végétales et animales et des dérivés, des huiles polaires préférées sont des esters, des éthers et des glycols et il est particulièrement préféré un poly(oxyde de propylène) tel que l'alpha-butoxy-polyoxypropylène.

8. Dispositif de collecte selon l'une quelconque des revendications 1 à 7, où le deuxième adhésif comprend en outre une résine poisseuse telle que des résines naturelles, modifiées ou synthétiques, de préférence des résines polaires telles que des esters de rosine et des dérivés de ceux-ci ou des résines de monomère aromatique pur.

9. Dispositif de collecte selon l'une quelconque des revendications précédentes, dans lequel le deuxième adhésif comprend des particules absorbantes.

10. Dispositif de collecte selon l'une quelconque des revendications précédentes, dans lequel le premier adhésif est à un emplacement adjacent à la source de déchets corporels.

11. Dispositif de collecte selon l'une quelconque des revendications précédentes, dans lequel le deuxième adhésif est situé au niveau du bord extérieur de la feuille gaufrée adhésive.

12. Dispositif de collecte selon l'une quelconque des revendications précédentes, dans lequel le deuxième adhésif est sous la forme d'un anneau.

13. Dispositif de collecte selon l'une quelconque des revendications précédentes, dans lequel le deuxième adhésif recouvre partiellement la feuille gaufrée adhésive.

14. Dispositif de collecte selon l'une quelconque des revendications précédentes, dans lequel l'épaisseur du deuxième adhésif est 400-600 $\mu$m, de préférence 500 $\mu$m.

15. Dispositif de collecte selon l'une quelconque des revendications précédentes, dans lequel le dispositif de collecte est un dispositif de stomie.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4192785 A
- WO 2009127208 A
- WO 2010074640 A
- DK 2008050146 W
- WO 02066087 A
- EP 1981554 A
- EP 1198261 A
- EP 2120812 A